# EUROPEAN PATENT APPLICATION

(11) **EP 4 767 968 A1**
(43) Date of publication of application: **01.07.2026**
(21) Application number: 25220701.4
(22) Date of filing: 04.12.2025
(51) Int. Cl.: A61B 18/14, A61B 5/287, A61B 18/12

(54) **CURVED END EFFECTOR FOR A MEDICAL CATHETER**

(30) Priority: 31.12.2024 US 202419007352
(71) Applicant: Biosense Webster (Israel) Ltd., 2066717 Yokneam (IL)
(72) Inventor: VAN NIEKERK, Pieter Emmelius, Irvine, 92618 (US); RODRIGUEZ SOTO, Juan, Irvine, 92618 (US); ABBAS, Mohammad, Irvine, 92618 (US); HIGHSMITH, Debby, Irvine, 92618 (US); EBRAHIMI, Babak, Irvine, 92618 (US)
(74) Representative: Carpmaels & Ransford LLP

(57) **Abstract**

The disclosed technology includes an end effector comprising a plurality of spines extending along a longitudinal axis to define a basket assembly, the plurality of spines configured to bow radially outward from the longitudinal axis to define a radius of curvature with respect to the longitudinal axis and to transition between an expanded configuration and a collapsed configuration. Each spine of the plurality of spines can include a section extending radially outward from the radius of curvature defined by a remainder of each spine. The end effector can further include at least one electrode disposed on the section for each spine of the plurality of spines.

## Description

### FIELD

The present invention relates generally to minimally invasive medical devices, and in particular sensing catheters, and further relates to, but not exclusively, cardiac mapping catheters and the manufacture thereof.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible end effectors designed to overcome this disadvantage. These catheters can include layered components that can be time-consuming, complex, and expensive to manufacture and assemble. Many times, electrode contact surfaces of these end effectors must be exposed through an encapsulating material via laser cutting or mechanical removal, both of which increase labor time and production costs.

### SUMMARY

To enable the time- and cost-efficient manufacture of end effectors that enhance the function of mapping and ablation catheters mentioned in the background section, the present disclosure relates to easily manufacturable end effectors, as well as fixtures and methods for manufacturing said end effectors having enhanced aspects of mapping and ablation catheter performance, including, but not limited to: mapping resolution, electrode contact with the target anatomy, delivery of the end effector to the target anatomy, biocompatibility, end effector stiffness, and atraumaticity.

There is provided, in accordance with the disclosed technology, an end effector having a length extending along a longitudinal axis from a proximal end to a distal end, a width extending along a horizontal axis perpendicular to the longitudinal axis, and a thickness extending along a vertical axis perpendicular to the longitudinal axis and the horizontal axis. The end effector can comprise a flexible circuit comprising a plurality of electrodes. The flexible circuit may be disposed on an insulative material and each electrode of the plurality of electrodes may include a contact surface. The insulative material may be contiguous to the contact surface so that only the contact surfaces of at least a portion of the plurality of electrodes are exposed to an ambient environment. A framework may be at least partially encapsulated in the insulative material and curved along the width in a direction along the vertical axis such that the end effector comprises a curvature along its width from about the proximal end to about the distal end.

The disclosed technology may include an end effector comprising a flexible circuit comprising a plurality of electrodes. The flexible circuit may be disposed within a first insulative layer of an insulative material and each electrode of the plurality of electrodes may include a contact surface. The insulative material may be contiguous to the contact surface so that only the contact surfaces of at least a portion of the plurality of electrodes are exposed to an ambient environment. A framework may be at least partially encapsulated in the first insulative layer and a lumen may be defined within the insulative material.

The disclosed technology may further include a method of manufacturing an end effector for a medical catheter. The method may comprise encapsulating a framework within a first insulative layer. The framework may comprise a shape-memory material configured to curve along a width of the framework from about a proximal end to about a distal end of the framework. The method may further include placing a flexible circuit comprising a plurality of electrodes on a first side of the first insulative layer, each electrode of the plurality of electrodes comprising a contact surface. The method can further include placing a sheet of insulative material in contact with the flexible circuit. The sheet of insulative material may be coupled to the outer surface of the first insulative layer to seal the flexible circuit between the sheet of insulative material and the first insulative layer.

Additional features, functionalities, and applications of the disclosed technology are discussed in more detail herein.

### BRIEF DESCRIPTION OF THE DRAWINGS

FIG. 1 is a schematic pictorial illustration of a medical system including a medical device, in accordance with the disclosed technology;
FIG. 2 is a schematic pictorial illustration of a perspective view of an end effector, in accordance with the disclosed technology;
FIG. 3 is a schematic pictorial illustration of an exploded perspective view of the end effector of FIG. 2;
FIG. 4 is a schematic pictorial illustration of a cross-section of the end effector, taken along line 4-4 in FIG. 2, in accordance with the disclosed technology;
FIG. 5A is a schematic pictorial illustration of a perspective view of an end effector, in accordance with the disclosed technology;
FIG. 5B is a schematic pictorial illustration of a perspective view of an end effector, in accordance with the disclosed technology;
FIG. 5C is a schematic pictorial illustration a perspective view of an end effector illustrating a radius of curvature, in accordance with the disclosed technology;
FIG. 5D is a schematic pictorial illustration a perspective view of an end effector illustrating a radius of curvature, in accordance with the disclosed technology;
FIG. 6 is a schematic pictorial illustration of a cross-section of an alternative configuration of an end effector, in accordance with the disclosed technology;
FIG. 7 is a schematic pictorial illustration of a cross-section of an alternative configuration of an end effector, in accordance with the disclosed technology;
FIG. 8A is a schematic pictorial illustration of a cross-section of an alternative configuration of an end effector, in accordance with the disclosed technology;
FIG. 8B is a schematic pictorial illustration of a cross-sectional detail view of FIG. 8A, in accordance with the disclosed technology;
FIG. 8C is a schematic pictorial illustration of a cross-section of an alternative configuration of an end effector, in accordance with the disclosed technology;
FIG. 8D is a schematic pictorial illustration of a cross-sectional detail view of FIG. 8C, in accordance with the disclosed technology;
FIG. 8E is a schematic pictorial illustration of a cross-sectional detail view of FIG. 8D, in accordance with the disclosed technology;
FIG. 9A is a schematic pictorial illustration of a cross-section of another alternative configuration of an end effector, in accordance with the disclosed technology;
FIG. 9B is a schematic pictorial illustration of a cross-section of another alternative configuration of an end effector, in accordance with the disclosed technology;
FIG. 10 a flowchart of a method of manufacturing an end effector for a medical device, in accordance with the disclosed technology;
FIG. 11 is a schematic pictorial illustration of a medical device assembly, in accordance with the disclosed technology; and
FIG. 12 is a schematic pictorial illustration of a top view of the end effector collapsed within a sheath, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator or any other individual or delivery instrumentation associated with delivery of a multi-electrode medical device for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells. For example, by utilizing thermal energy, such as radio frequency (RF) ablation, or non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, methods or uses and devices for mapping and ablation of cardiac tissue to treat cardiac arrhythmias. Ablative energies are typically provided to cardiac tissue by a tip portion of a medical device (e.g., a medical probe or catheter) which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

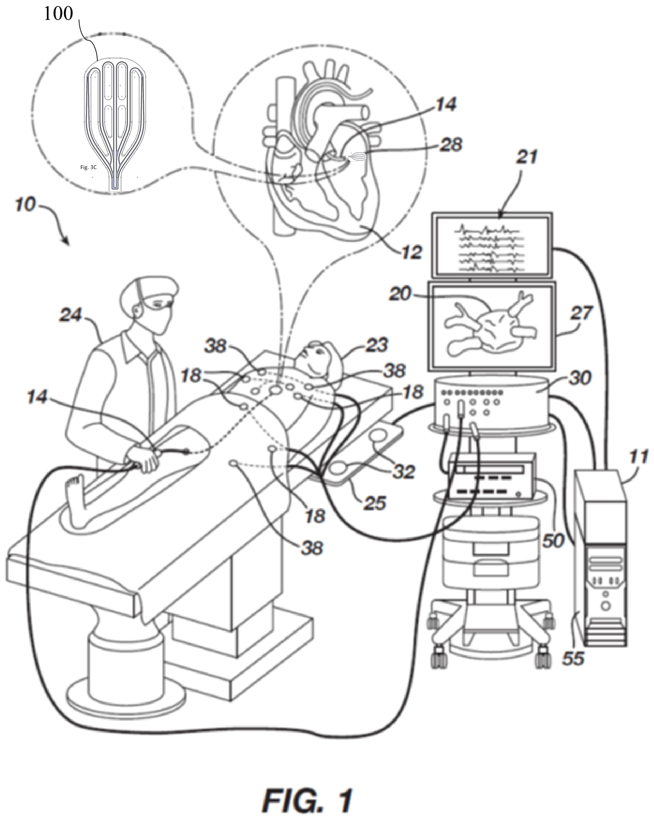

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a catheter shaft with distal tip 28 of catheter 14 (i.e., multilayered end effector 100) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes optionally distributed over end effector 100 coupled to a catheter shaft and configured to sense the IEGM signals as described in more detail below. Catheter 14 may additionally include a position sensor embedded in or near end effector 100 for tracking position and orientation of end effector 100. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes. For impedance-based tracking, electrical current is directed toward the electrodes and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes of the catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26 at a distal tip 28 of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

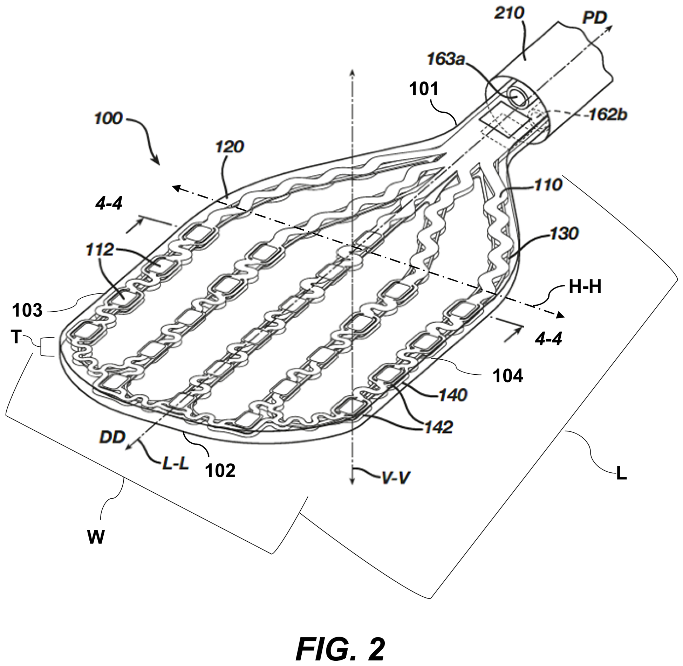

FIG. 2 illustrates an end effector 100 in accordance with an example of the present disclosure in order to achieve the ease of manufacturing, reduced cost, and enhanced end effector properties, such as desired collapsibility, maneuverability, robustness, stiffness, mapping resolution, electrode contact with target anatomy, and conformity of the end effector 100 disclosed herein to flat, curved, irregular and/or nonplanar tissue surfaces found in the target anatomy. The end effector 100 may comprise a length L extending along a longitudinal axis L-L from a proximal end to a distal end, a width W extending along a horizontal axis H-H perpendicular to the longitudinal axis, and a thickness T extending along a vertical axis V-V perpendicular to the longitudinal axis and the horizontal axis.

The end effector 100 can include a flexible circuit 110 including a plurality of electrodes 112, each electrode of the plurality of electrodes 112 including a contact surface 112c. As used herein, the term "flexible circuit" includes thin-film circuit, flexible printed circuit board, thin film deposition via lithography and etching processes on substrate such as polyimide, copper, LCP (e.g., Panasonic FELIOS^{™} SERIES of Flexible Circuit Boards), nitinol substrate or directly onto the polymeric substrate such as Panasonic BEYOLEX^{™} PRINTED ELECTRONICS SUBSTRATE Series. In some examples, the flexible circuits described herein can be made primarily of polyimide. In other examples, it can be made of any of biocompatible polyimides, glass-reinforced epoxy laminate materials, copper, or graphene, alone or in combination. The first 110 and second 140 flexible circuits can include conductive traces directly formed onto the polymeric substrate with the electrode comprising a substantially much thicker version of the traces such that the typical polyimide substrate is no longer required. In some examples, the electrodes described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The flexible circuit 110 can be disposed on an insulative material 120 (also known as an insulative mass). The insulative material 120 can be contiguous to the contact surfaces 112c so that only the contact surfaces 112c of at least a portion of the plurality of electrodes 112 are exposed to the ambient environment. As used herein, the term "contact surface" includes the portion of an electrode having a generally flat surface and the edge or edges which immediately surround said flat surface. Electrodes 112 may have a slightly rounded, radiused, or chamfered edge that comes into contact with tissue, along with the generally flat surface, when the end effector 100 is placed against tissue. As used herein, "ambient environment" refers to the external environment such as the organ in which the end effector 100 is deployed or in the operating theater prior to being deployed in the biological organ.

It is noted that not all of the electrodes 112 on the end effector 100 described herein need be exposed through the insulative material 120 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood. Irrigation can be provided with irrigation ports 163a on one side and 162b on the other side in fluid communication with an irrigation line not shown disposed in a catheter shaft 210. Instead of an irrigation line separate from the catheter shaft 210, a lumen can be formed via extrusion of the catheter shaft 210 to provide for a lumen channel. It is noted that port 163a or 162b can be configured to have a sufficient flow diverter characteristic for irrigation fluid to cover the mapping electrodes during irrigation flow so as to prevent or reduce thrombus formations.

The end effector 100 can further include a framework 130 contiguous to the insulative material 120 or at least partially encapsulated in the insulative material 120. In examples in which the end effector 100 includes framework 130, the framework 130 can be spaced from the flexible circuit 110 by some of the insulative material 120 coming therebetween.

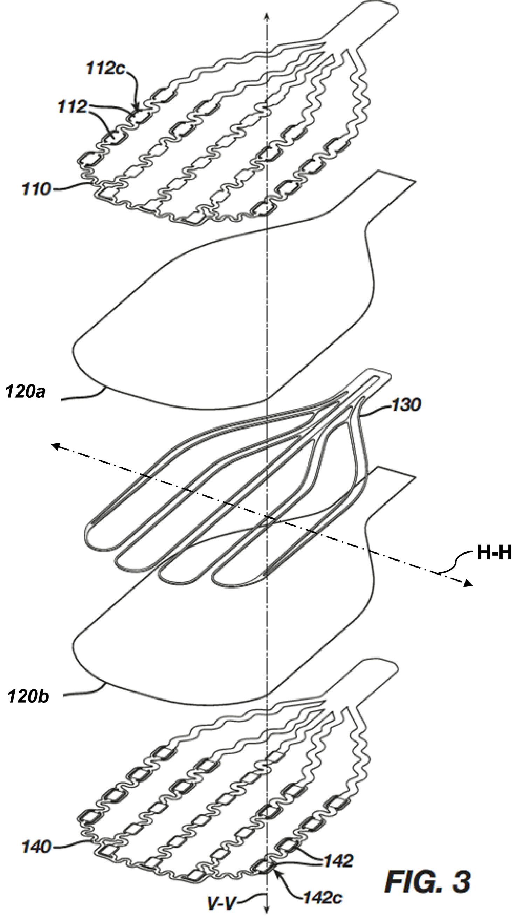

FIG. 3 shows an exploded view of the end effector 100, with the components thereof exploded vertically along vertical axis V-V (also referred to as the height direction) of the end effector 100. The flexible circuit 110 can be a first flexible circuit 110 and the plurality of electrodes 112 can be a plurality of first electrodes 112, with each first electrode 112 including a first contact surface 112c. The end effector 100 can further include a second flexible circuit 140 having a plurality of second electrodes 142. The second flexible circuit 140 can be spaced apart from the first flexible circuit 110 and each electrode of the second plurality of electrodes 142 can have a second contact surface 142c. The contact surfaces 112c, 142c, in use, are configured to either contact or be disposed proximal to tissue. Moreover, each flexible circuit 110, 140 includes respective tines extending along the longitudinal axis L-L, with the tines supporting the respective electrodes.

In examples having first 110 and second 140 flexible circuits, the insulative material 120 can be disposed between the first flexible circuit 110 and the second flexible circuit 140, and the insulative material 120 can be contiguous to the second contact surfaces 142c so that only the contact surface 142c of each second electrode 142 is exposed to the ambient environment, similar to how first electrodes 112 are disposed in and exposed through the insulative material 120.

Electrodes 112, 142 can sense tissue signals or transmit energy AC or DC from an energy generator to the tissues. In some examples, there are about 92 electrodes. In some examples, there are about 48 electrodes. In some examples, there are about 64 electrodes. In some examples, there are about 72 electrodes. In some examples, there are about 98 electrodes. However, those skilled in the art will appreciated that various numbers of electrodes 112, 142 can be employed without departing from the spirit and scope of the present disclosure.

In examples herein, the end effector 100 includes a framework 130 disposed between the first flexible circuit 110 and the second flexible circuit 140. Framework 130 can be a component of the end effector 100 that is separate and distinct from the first flexible circuit 110 and disposed proximate the first flexible circuit 110. In this case, the insulative material 120 can be further disposed between the framework 130 and the second flexible circuit 140. The framework 130 can be formed from a planar or cylindrical stock of material using any suitable method. For example, the framework 130 can be formed by cutting, laser cutting, stamping, etc.

The framework includes a plurality of spines extending along the longitudinal axis L-L. The tines of the flexible circuits 110, 140 can be approximately aligned with one or more of the spines such that the spines 132, provide support thereto.

Insulative material 120 can include a first sheet of insulative material 120a and a second sheet of insulative material 120b fused together proximate the framework 130 into a single, contiguous, generally planar insulative material 120. This insulative material 120 also serves to enhance the atraumaticity of the end effector 100 and to protect the subject from sharp edges.

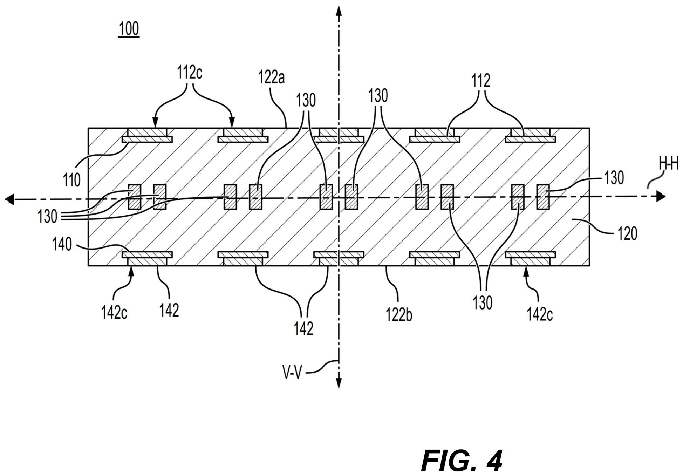

FIG. 4 depicts a cross-sectional view of the end effector 100. As seen in FIG. 4, the insulative material 120 includes opposing outer surfaces 122a (referred to herein as a first insulative surface 122a), 122b (referred to herein as a second insulative surface 122b). In some examples, the first and second outer surfaces 122a, 122b extend in approximately parallel planes.

The insulative material 120 can include polymer. The insulative material 120 can be heat formed around at least a portion of the first flexible circuit 110, the second flexible circuit 140, and the framework 130. The polymer can include thermoplastic polyurethane (TPU) or other heat formed or shaped material which lends itself to said heat forming, such as, but not limited to, silicone or siloxane.

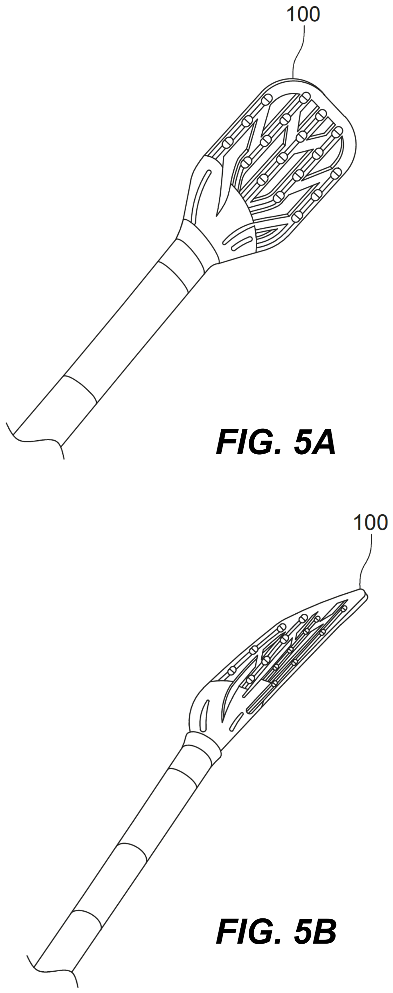

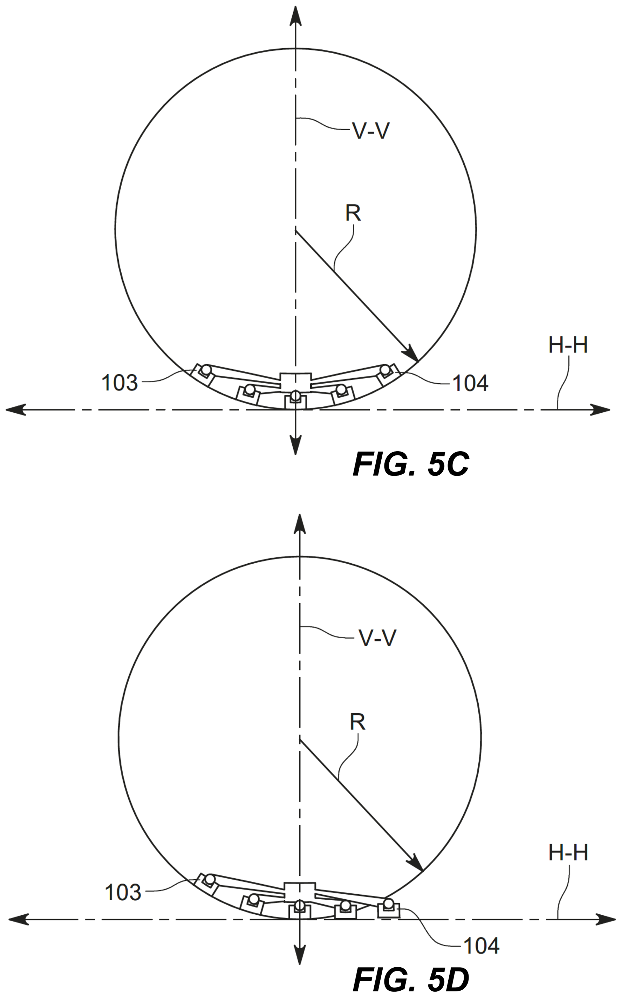

FIGS. 5A-5D depict a curved end effector, according to some examples. In some examples, framework 130 may be curved along the width W, relative to a horizontal axis H-H, in a direction along the vertical axis such that the end effector 100 will have a curvature along its width W from the proximal end to about the distal end. The curvature may assist in reducing the insertion force, and reduce shearing risks.

In some embodiments, as depicted by FIG. 5C the curvature may be uniform throughout length of paddle. In some examples, the curvature may comprise a radius R of about 8 millimeters (mm) to about 15 mm. In some examples, the curvature can start at an initial radius, for example, about 4 mm at a first end 103, and gradually open to a flat paddle at a second end 104. The curvature may be provided to facilitate contact of the electrodes of the end effector 100 onto a tissue surface to be treated.

In some embodiments, the curvature may be applied along the length L of the end effector, relative to the longitudinal axis L-L, in a direction along the horizontal axis H-H. Similar to a curvature along the width W, a curvature along the length may comprise a constant radius or an increasing radius. For example, the curve can start at an initial radius, for example, 4 mm at the proximal end 101, and gradually open to a flat paddle at the distal end 102. In some examples, the end effector may be curved along both its width W and length L.

In some examples, the curve can be created by a shape set in the framework 130. In some examples, the curve can be created by securing a flat framework 130 in a curved configuration to a component such as an insert, tubing, or a tether. In some examples, the framework 130 comprises a shape-memory material. In some examples, the shape-memory material configured to return to a predefined shape having the curvature at an activation temperature. This may facilitate movement of the end effector 100 through a sheath before being activated to return to a set shape within a body of a patient. In some examples, the activation temperature of the shape memory material is about 37 degrees Celsius. The shape-memory material may comprise nitinol or similar biocompatible shape memory materials such as copper-aluminum-nickel alloy, titanium-niobium alloy, titanium-tantalum alloy, titanium-molybdenum alloy, titanium-zirconium alloy, or the like.

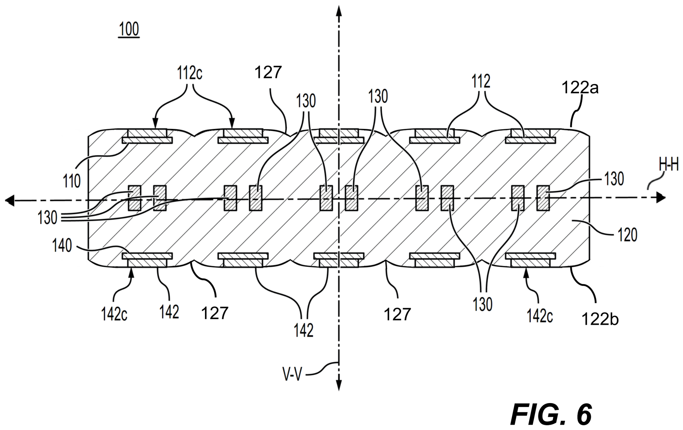

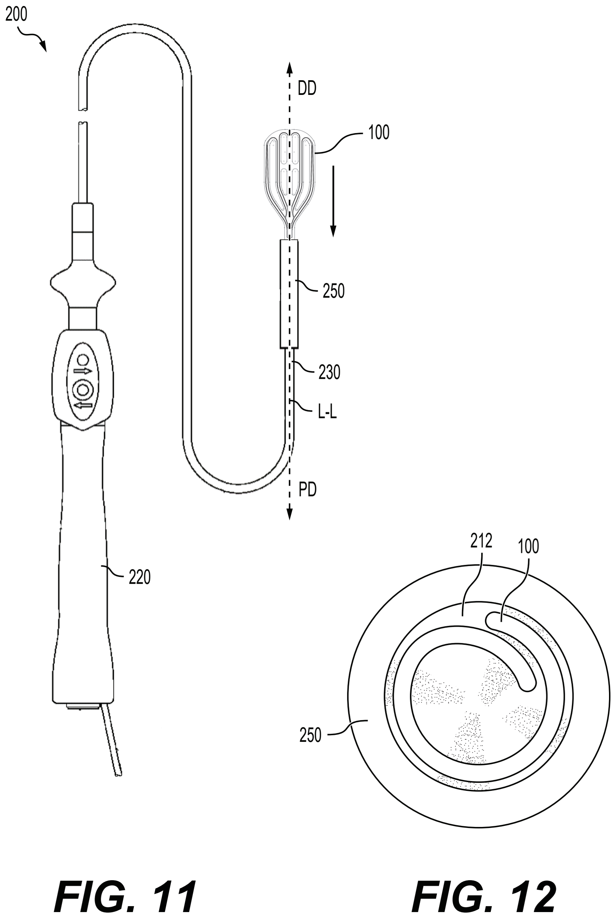

With reference to FIG. 6, in some examples, an outer surface 122a, 122b of the insulative material 120 may comprise a plurality of creases 127 along the length L of the end effector 100. The creases 127 may facilitate folding or rolling of the end effector 100 when traveling through or being inserted into a sheath, as depicted in FIG. 12. The creases 127 may provide predetermined fold and crumple lines or zones. The creases 127 may reduce stress concentrations in the insulative material 120 or critical components (electrical traces, etc.) as the end effector is folded to prevent fracturing or damage to the insulative material 120. In some examples, the creases 127 are provided on the outer surfaces 122a, 122b of the insulative material 120. In some examples, the creases 127 may only be provided on one side of the end effector, such that only a first outer surface 122a or a second outer surface 122b of the insulative material 120 comprises the creases 127, and the opposing outer surface is substantially flat. Creases 127 added on one side to may promote folding in a specific direction, at specific locations. In some examples, the forming the creases 127 comprises at least one of laser drilling, chemical etching, or stamping. In some examples, a separate component with creases 127 (e.g., a creased polyimide layer) can be laminated to be part of the end effector assembly.

In some examples, insulative material 120 can be shaped, scalloped, ribbed, ridged, concaved, convexed, or otherwise configured such that the overall profile of insulative material 120 yields physical and/or mechanical properties, such as rigidity and flexion along multiple axes, required by the end effector 100, mentioned above.

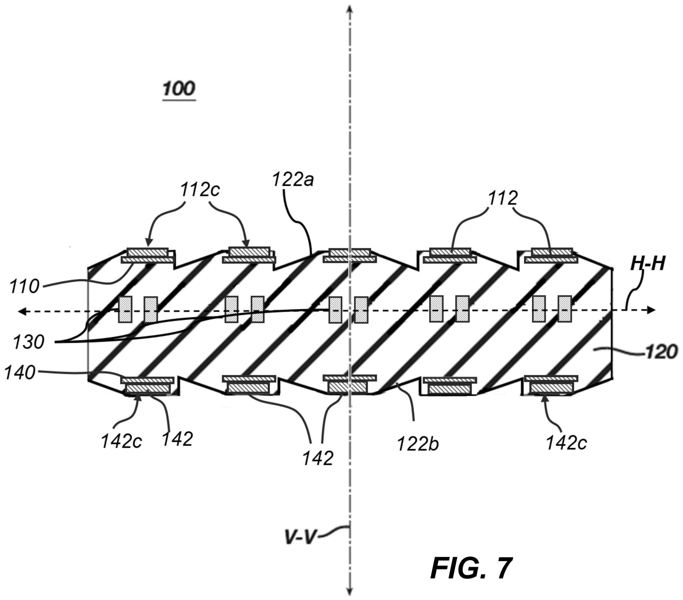

With reference to FIG. 7, in some examples, the outer surfaces 122a, 122b of the insulative material 120 may comprise slanted a plurality of slanted edges. In some examples, as depicted in FIG. 11, a medical probe assembly 200 includes a tubular member 230 extending along a longitudinal axis L-L and is configured to deliver any of the end effectors 100 previously discussed to, out of, and/or back into a sheath 250. As seen particularly in FIG. 12, as the end effector 100 collapses into lumen 212 of sheath 250, the sides of the end effector 100 may meet and compresses against each other until one side slips inward, causing the paddle can roll on itself. Providing slants at the edges can promote slipping which may facilitate earlier rolling of the end effector 100, thereby decreasing resistance as the end effector 100 collapses into a sheath or insertion tool. In some embodiments, the slanted edges are imprinted with a lamination fixture.

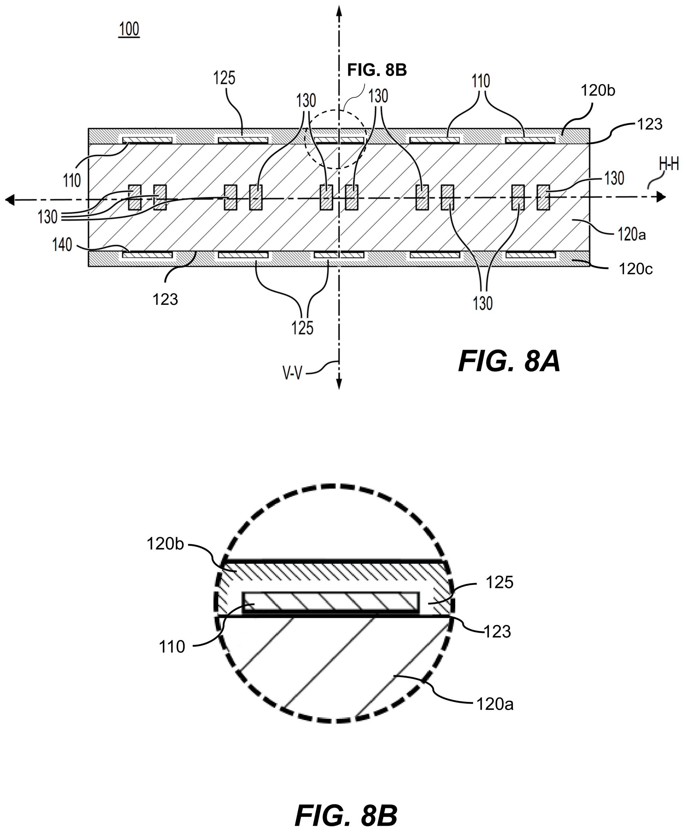

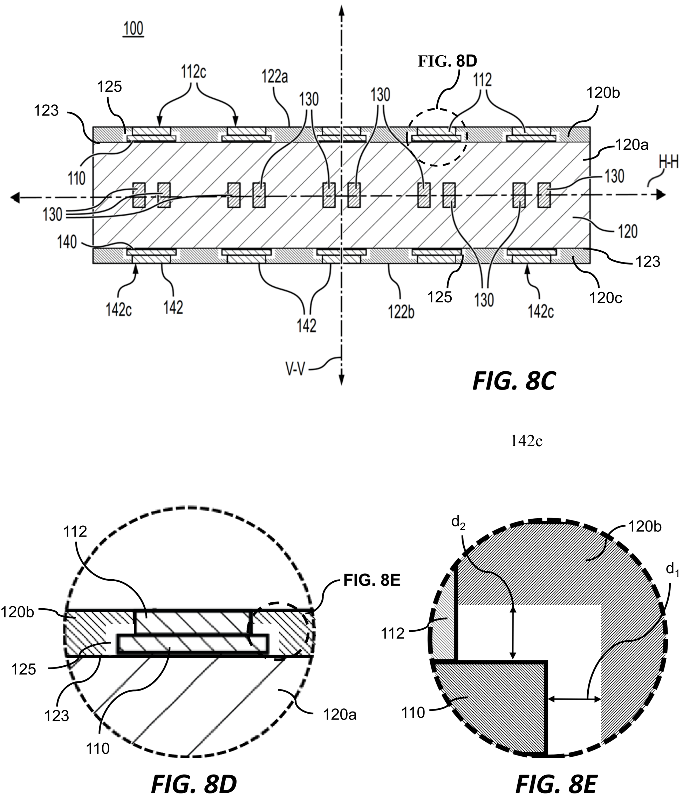

FIGS. 8A-8E depict cross-sectional views of a multilayered end effector 100 that includes a first flexible circuit 110, first electrodes 112, insulative material, a framework 130, a second flexible circuit 140, and second electrodes 142. The insulative material encapsulates and/or spaces the components of the end effector 100, as discussed in previous examples. In this configuration, the insulative material is formed from three insulative sheets: a first sheet 120a, a second sheet 120b, and a third sheet 120c. A boundary layer 123 is formed where the second sheet 120b abuts the first sheet 120a, and wherein the third sheet 120c abuts the first sheet 120a. The framework 130 is at least partially encapsulated with the first sheet 120a of insulative material, the first flexible circuit 110 is at least partially encapsulated with the second sheet 120b of insulative material, and the second flexible circuit 140 is at least partially encapsulated with the third sheet 120c of insulative material. The flexible circuits 110, 140 may be provided on or adjacent to the boundary layers 123.

In some examples, a gap 125 between at least one surface of the flexible circuits 110, 140, and the respective sheets 120b, 120c of insulative material is provided. The gaps 125 provide a separation between the flexible circuits 110, 140 and the insulative material provide additional flexibility of the end effector 100, for example as the end effector 100 is bent relative to a horizontal axis H-H.

With reference to FIG. 8A, the gaps 125 may be provided between at least one surface of the flexible circuits 110, 140 along the length of the end effector 100. In some examples, the gaps 125 are provided around three surfaces, a top surface, and two sides of the flexible circuits 110, 140. As depicted, in FIG. 8E, a separation distance d₁ may be provided between the sides of the flexible circuits and the insulative material and a separation distance d₂ may be provided between a top surface of the flexible circuit and the insulation material. In some examples, the separation distance d₁ the separation distance d₂ are equal. FIG. 8C depicts a cross-section view at a location where electrodes 112, 142 are provided on the flexible circuits, wherein gaps 125 are provided around the flexible circuits 110, 140 but the insulative material may abut the electrodes 112, 114. While the figures depict flexible circuits 110, 140 having rectangular cross-sections, one can appreciate that similar gaps 125 could be applied to flexible circuits having alternative shapes.

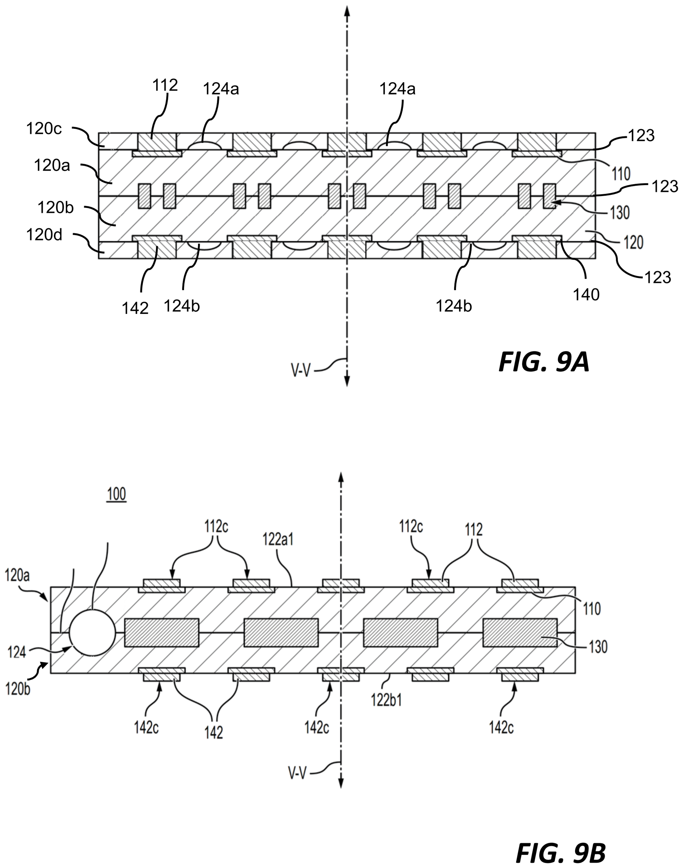

FIGS. 9A and 9B depict other aspects of the present disclosure. Irrigation is typically required in medical catheters for various reasons, such as, but not limited to, for cooling and/or temperature control (e.g., during ablation) and for the prevention of clot formation. The designs illustrated in these figures can be employed with similar (or the same) concepts as previously described (e.g., creases, a curved end effector, flexible circuits 110, 140, and electrodes 112, 142). In some examples, at least one lumen is formed within the insulative material 120.

FIG. 9A depicts a cross-sectional view of a multilayered end effector 100 that includes a first flexible circuit 110, first electrodes 112, insulative material 120, a framework 130, a second flexible circuit 140, and second electrodes 142. The insulative material 120 similarly encapsulates and/or spaces the components of the end effector 100, as discussed in previous examples. In this configuration, the insulative material 120 is formed from four insulative sheets: a first sheet 120a, a second sheet 120b, a third sheet 120c, and a fourth sheet 120d that are heat formed together to form one contiguous mass. In some examples, respective boundary layers 123 are formed where the insulative sheets abut one another. The framework 130 is disposed on and sandwiched by and the first insulative sheet 120a the second insulative sheet 120b. The flexible circuits 110, 140 are shown as being disposed on the third insulative sheet 120c and the fourth insulative sheet 120d, respectively, but, as will be appreciated by those skilled in the art, the flexible circuits 110, 140 can be disposed on any appropriate layer/sheet of the insulative material 120 without departing from the spirit and scope of the present disclosure.

The insulative material 120 defines one or more first lumens 124a that are formed between the adjacent first and third sheets 120a, 120c. Depending on the irrigation requirements of the system, one or more second lumens 124b can additionally be formed between the adjacent second and fourth sheets 120b, 120d. These lumens 124a, 124b can extend from a proximal end of the end effector 100 to the distal end thereof and/or include channels formed through the outermost sheets 120c, 120d at predetermined locations along the longitudinal axis L-L. Methods of forming these lumens 124, 126 are discussed in greater detail herein.

In some examples, the one or more first lumens 124a are formed by recesses provided in the third sheet 120c such that the first lumens 124a are defined at a boundary layer 123 wherein the first insulative sheet 120a and the third insulative sheet are joined. Similarly, the one or more second lumens 124a may be formed by recesses provided in the fourth sheet 120d such that the second lumens 124b are defined at a boundary layer 123 wherein the second insulative sheet 120b and the fourth insulative sheet are joined.

FIG. 9B depicts as similar concept as that of FIG. 9A, but with a different configuration of insulative material 120 and a lumen 124 that demonstrates how an alternative approach that employs the concepts discussed herein. In this example, a first sheet 120a and a second sheet 120b formed together to form the insulative material/mass 120 at least partially encapsulating the framework. A single lumen 124 is formed by recesses provided in the first sheet 120a and the second sheet 120b collectively define the lumen 124 when formed together. In this example, the lumen 124 is positioned laterally relative to the framework 130 and flexible circuits 110, 140. An additional insulative sheet may be provided on the outer surface 122a1 of the first sheet 120a to partially encapsulate the electrodes 112. Similarly, an additional insulative sheet may be provided on the outer surface 122b1 of the second sheet 120a to partially encapsulate the electrodes 142.

As discussed herein, insulative sheets can be joined by any suitable process such as, for example, thermal, mechanical, or laser formation processes. In some examples, the lumens can be formed by selectively leaving one or more regions of the insulative material 120 unlaminated (i.e., not heat formed or laser welded), leaving some areas not laminated provides tunnels for irrigation fluid. In some examples, the lumen is formed by selectively laminating the first sheet and the second sheet such that a non-laminated section of the first sheet and the second sheet defines the lumen. For example, the end effector 100 of FIG. 9A can have the lumens 124a created by selectively laminating the sheets 120a, 120c (e.g., via laser welding) at their abutting surfaces 123 with non-laminated sections forming voids that function as irrigation lumens 124a.
As discussed herein, laser welding may be used to selectively bond various layers of a TPU/thermoplastic polymer matrix which form the insulative sheets. This may allow for complete sealing of components within the polymer matrix, while also affording the flex circuit some freedom to move/shift. In other examples, a heat press with dedicated hot zones (through bossing, cooling lines, various conductive and non-conductive materials) can also be used for selective lamination, leaving one or more regions of the insulative material 120 unlaminated (i.e., not heat formed) thereby providing tunnels for irrigation fluid

In some examples, the lumen is formed by forming a recess in one of the sheets (e.g., the first sheet). This can be done by any appropriate process, such as laser cutting.

In some examples, the lumen is formed, in part, by positioning a second material with a higher melting point than the insulative material between the first sheet and the second sheet. By way of example, the second material can include, but is not limited to polytetrafluoroethylene (PTFE), ethylene tetrafluoroethylene (ETFE), polyimide, and the like. Besides tubular shapes, two or more flat/rectangular strips can be positioned with a gap therebetween to form a rectangular shaped lumen.

In some examples where a second material is employed (e.g., polyether ether ketone (PEEK), polished stainless steel or the like), the two sheets can be laminated and the second material removed thereafter, leaving a void as the lumen. In other words, the second material is only temporarily a part of the end effector 100 structure as a portion of the manufacturing process.

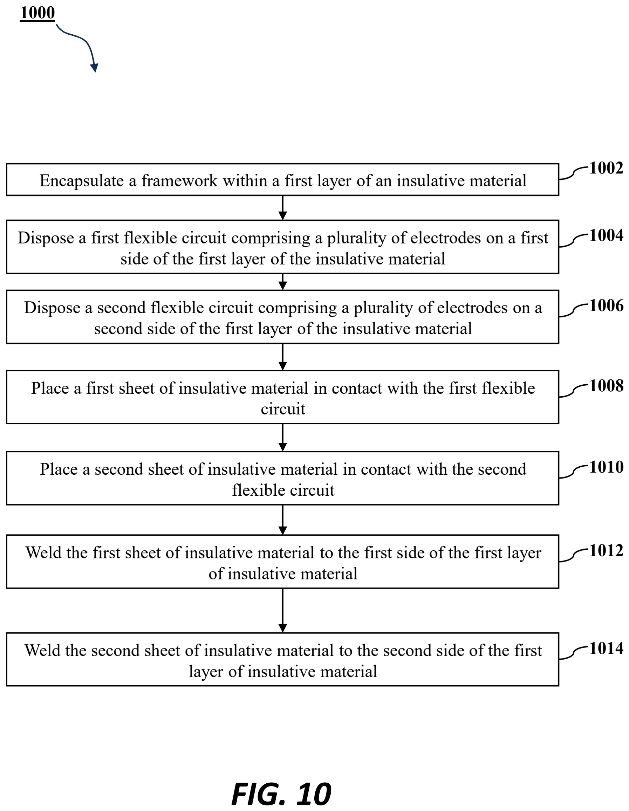

FIG. 10 depicts an exemplary method 1000 of manufacturing an end effector 100 for a medical catheter. In some examples, the method comprises encapsulating a framework 130 within a first insulative layer at step 1002. In some examples, a first flexible circuit is comprising a plurality of electrodes is disposed on a first side of the first insulative layer at step 1004. In some examples, the method further comprises placing a sheet of insulative material in contact with the first flexible circuit at step 1008. In some examples, the sheet of insulative material is coupled to the outer surface of the first insulative layer to seal the first flexible circuit between the sheet of insulative material and the first insulative layer at step 1012. The method may further comprise disposing a second flexible circuit is comprising a plurality of electrodes is disposed on a second side of the first insulative layer at step 1006. In some examples, the method further comprises placing a second sheet of insulative material in contact with the second flexible circuit at step 1010 In some examples, a second sheet of insulative material is coupled to the first insulative layer to seal the second flexible circuit between the second sheet of insulative material and the first insulative layer at step 1014. The method may further comprise a step of exposing the contact surface of each electrode 112 by laser cutting the sheet of insulative material around the contact surface 112c of each electrode 112.

The disclosed technology described herein can be further understood according to the following clauses:
Clause 1: An end effector having a length extending along a longitudinal axis from a proximal end to a distal end, a width extending along a horizontal axis perpendicular to the longitudinal axis, and a thickness extending along a vertical axis perpendicular to the longitudinal axis and the horizontal axis, the end effector comprising: a flexible circuit comprising a plurality of electrodes, the flexible circuit disposed on an insulative material and each electrode of the plurality of electrodes comprising a contact surface, the insulative material being contiguous to the contact surface so that only the contact surfaces of at least a portion of the plurality of electrodes are exposed to an ambient environment; and a framework at least partially encapsulated in the insulative material and curved along the width in a direction along the vertical axis such that the end effector comprises a curvature along its width from about the proximal end to about the distal end.
Clause 2: The end effector of Clause 1, the curvature comprising a radius of about 8 millimeters to about 15 millimeters.
Clause 3: The end effector of Clause 1, the radius of the curvature being uniform throughout the length of the end effector.
Clause 4: The end effector of Clause 1, the radius of the curvature increasing from the proximal end to the distal end.
Clause 5: The end effector of Clause 4, the radius of the curvature at the proximal end being approximately 4 millimeters.
Clause 6: The end effector of Clause 1, the framework comprising a shape-memory material configured to return to a predefined shape having the curvature at an activation temperature.
Clause 7: The end effector of Clause 6, the activation temperature being about 37 degrees Celsius.
Clause 8: The end effector of Clause 6, the shape-memory material comprising nitinol.
Clause 9: The end effector of Clause 1, an outer surface of the insulative material comprising a plurality of creases along the length of the end effector.
Clause 10: The end effector of Clause 1, an outer surface of the insulative material being slanted between adjacent spines of the framework.
Clause 11: The end effector of Clause 1, the insulative material further defining a gap between at least one surface of the flexible circuit and an interior surface of the insulative material.
Clause 12: The end effector of Clause 1, further comprising a lumen formed within the insulative material.
Clause 13: The end effector of Clause 12, the lumen disposed adjacent to spines of the framework.
Clause 14: A method of manufacturing an end effector for a medical catheter, the method comprising: encapsulating a framework within a first insulative layer, the framework comprising a shape-memory material configured to curve along a width of the framework from about a proximal end to about a distal end of the framework; placing a flexible circuit comprising a plurality of electrodes on a first side of the first insulative layer, each electrode of the plurality of electrodes comprising a contact surface; placing a sheet of insulative material in contact with the flexible circuit; and coupling the sheet of insulative material to the outer surface of the first insulative layer to seal the flexible circuit between the sheet of insulative material and the first insulative layer.
Clause 15: The method of Clause 14, further comprising a step of exposing the contact surface of each electrode by laser cutting the sheet of insulative material around the contact surface of each electrode.
Clause 16: The method of Clause 14, the coupling of the sheet of insulative material to the outer surface of the first insulative layer comprising laser coupling.
Clause 17: The method of Clause 14, the coupling of the sheet of insulative material to the outer surface of the first insulative layer comprising thermal coupling.
Clause 18: The method of Clause 14, the end effector defining a gap between a point at which the sheet of insulative material is welded to the outer surface and the flexible circuit.
Clause 19: The method of Clause 14, further comprising forming creases in the sheet of insulative material.
Clause 20: The method of Clause 19, the forming of the creases comprising at least one of laser drilling, chemical etching, or stamping.
Clause 21: The method of Clause 14, further comprising forming a lumen within the first insulative layer.
Clause 22: The method of Clause 14, further comprising forming a first lumen between the first insulative layer and the sheet of insulative material.
Clause 23: An end effector comprising: a flexible circuit comprising a plurality of electrodes, the flexible circuit disposed within a first insulative layer of an insulative material and each electrode of the plurality of electrodes comprising a contact surface, the insulative material being contiguous to the contact surface so that only the contact surfaces of at least a portion of the plurality of electrodes are exposed to an ambient environment; a framework at least partially encapsulated in the first insulative layer; and a lumen defined within the insulative material.
Clause 24: The end effector of Clause 23, the lumen being formed within the first insulative layer.
Clause 25: The end effector of Clause 23, further comprising a first sheet of insulative material attached to the first insulative layer, the lumen being formed between the first insulative layer and the first sheet of insulative material.
The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. An end effector having a length extending along a longitudinal axis from a proximal end to a distal end, a width extending along a horizontal axis perpendicular to the longitudinal axis, and a thickness extending along a vertical axis perpendicular to the longitudinal axis and the horizontal axis, the end effector comprising:
a flexible circuit comprising a plurality of electrodes, the flexible circuit disposed on an insulative material (120) and each electrode of the plurality of electrodes comprising a contact surface, the insulative material being contiguous to the contact surface so that only the contact surfaces of at least a portion of the plurality of electrodes are exposed to an ambient environment; and
a framework at least partially encapsulated in the insulative material and curved along the width in a direction along the vertical axis such that the end effector comprises a curvature along its width from about the proximal end to about the distal end.

2. The end effector of claim 1, the curvature comprising a radius (R) of about 8 millimeters to about 15 millimeters.

3. The end effector of claim 1, the radius of the curvature being uniform throughout the length of the end effector.

4. The end effector of claim 1, the radius of the curvature increasing from the proximal end to the distal end, optionally the radius of the curvature at the proximal end being approximately 4 millimeters.

5. The end effector of claim 1, the framework comprising a shape-memory material configured to return to a predefined-shape having the curvature at an activation temperature.

6. The end effector of claim 5, the activation temperature being about 37 degrees Celsius or the shape-memory material comprising nitinol.

7. The end effector of claim 1, an outer surface of the insulative material i) comprising a plurality of creases along the length of the end effector or ii) being slanted between adjacent spines of the framework.

8. The end effector of claim 1, the insulative material further defining a gap between at least one surface of the flexible circuit and an interior surface of the insulative material.

9. The end effector of claim 1, further comprising a lumen formed within the insulative material, optionally the lumen disposed adjacent to spines of the framework.

10. A method of manufacturing an end effector for a medical catheter, the method comprising:
encapsulating a framework within a first insulative layer, the framework comprising a shape-memory material configured to curve along a width of the framework from about a proximal end to about a distal end of the framework;
placing a flexible circuit comprising a plurality of electrodes on a first side of the first insulative layer, each electrode of the plurality of electrodes comprising a contact surface;
placing a sheet of insulative material in contact with the flexible circuit; and
coupling the sheet of insulative material to the outer surface of the first insulative layer to seal the flexible circuit between the sheet of insulative material and the first insulative layer.

11. The method of claim 10, further comprising a step of exposing the contact surface of each electrode by laser cutting the sheet of insulative material around the contact surface of each electrode.

12. The method of claim 10, the coupling of the sheet of insulative material to the outer surface the first insulative layer comprising laser coupling.

13. The method of claim 10, the end effector defining a gap between a point at which the sheet of insulative material is welded to the outer surface and the flexible circuit.

14. The method of claim 10, further comprising forming creases in the sheet of insulative material.

15. The method of claim 10 further comprising forming a lumen within the first insulative layer, or forming a first lumen between the first insulative layer and the sheet of insulative material.
